# EUROPEAN PATENT SPECIFICATION

(11) **EP 2 760 509 B1**
(45) Date of publication and mention of the grant of the patent: **05.04.2023**
(21) Application number: 12766092.6
(22) Date of filing: 27.09.2012
(51) Int. Cl.: A61M 5/31, A61M 5/315

(54) **USE OF PLASMA TREATED SILICONE OIL AS A COATING IN A MEDICAL INJECTION DEVICE**
VERWENDUNG VON PLASMABEHANDELTEM SILIKONÖL ALS BESCHICHTUNG IN EINER MEDIZINISCHEN INJEKTIONSVORRICHTUNG
UTILISATION D'HUILE DE SILICONE TRAITÉE AU PLASMA COMME REVÊTEMENT DANS UN DISPOSITIF MÉDICAL D'INJECTION

(30) Priority: 27.09.2011 EP 11306236
(43) Date of publication of application: 06.08.2014
(60) Divisional application: 23157103.5
(73) Proprietor: Becton Dickinson France, 38800 Le Pont-de-Claix (FR)
(72) Inventor: JANVIER, Sébastien, 38000 Grenoble (FR); MANGIAGALLI, Paolo, 38120 Fontanil (FR); JOUFFRAY, Sébastien, 38000 Grenoble (FR); FOUCHER, Cédric, 38000 Grenoble (FR); ENFOUX, Yves, 38760 Varces (FR)
(74) Representative: Regimbeau
(86) International application number: PCT/EP2012/069119
(87) International publication number: WO 2013/045571

(56) References cited:
- EP-A2- 0 920 879
- WO-A1-2010/034004
- WO-A1-2011/092536
- JP-A- 2005 160 888
- US-A1- 2008 071 228
- US-B1- 6 461 334

## Description

### FIELD OF THE INVENTION

The invention relates to the use of a plasma treated silicone oil layer as a coating for a medical injection device comprising a barrel and a stopper in a gliding engagement within the barrel, for limiting the number of particles released in a pharmaceutical composition contained in the injection device.

### TECHNICAL BACKGROUND

Medical injection devices that comprise a sealing stopper in a gliding engagement within a container are widely used to deliver drug by injection to a patient.

Such injection devices include syringes, cartridges but also auto-injectors.

Many different types of injection devices have been designed for administering medicines. Injection devices usually comprise a container intended to receive the product to be injected and a plunger rod intended to move a stopper within the container so as to expel the product therefrom at the time of injection. Empty and pre-filled disposable injection devices exist but prefilled devices are now preferred because they are more convenient, safe and efficient and may be used directly in emergency cases.

Such injection devices would be appropriate for containing the new high-value drugs that are commonly named "biotech" drugs, and that are currently developed and launched on the pharmaceutical market.

These biotech drugs are made out of living cell cultures, instead of the simple molecules used to create traditional pharmaceuticals, and can comprise for example biological elements such as proteins, peptides, DNA, RNA and the like, as well as sensitive drugs.

A major constraint with such drugs is their sensitivity towards their environment and therefore there is a need to find an appropriate container able to maintain the integrity of the drugs when they are stored for a long time in injections devices.

Traditional injections devices comprise a container made of glass or plastic and a stopper made of rubber.

Optionally, coatings can be applied on the surface of the container and/or on the surface of the stopper in order to improve the lubrication of the injection device.

But in some cases, it has been shown that such injections devices are not appropriate for long storage of high-value drugs due to the high and long interaction with their containers.

Indeed, it has been shown that some degradations of the drug (aggregation, denaturing, etc...) occur with time, leading to the rejection of the prefilled device and therefore leading to a high loss for pharmaceutical companies.

Therefore there is a need to have a prefilled injection device having a medical container compatible with biotech drugs, meaning a container that avoids any denaturing and/or aggregation of the drug

Besides, such prefilled injection devices should also have good gliding properties i.e. a low gliding force to move the stopper within the container.

Moreover, if a coating is applied on the surface of the prefilled injection device, the integrity of the coating layer has to be maintained from the deposition of the layer on the surface(s) of the medical device until the injection of the drug to the patient.

It means that any delaminating or breaking phenomena of the layer are to be avoided.

This is particularly the case for lubrication layers that should be required to enhance the gliding properties of the injection devices.

Finally, the integrity of the drug, the integrity of the coating and the gliding properties of the prefilled device need to be maintained over time i.e. to be stable for at least the shelf life of the prefilled injection device.

Some documents such as US 5,338,312 and EP 0 201 915 describe an injection device having enhanced gliding properties by using a lubricant layer that is crosslinked in order to immobilize the layer on the surface of the container of the injection device.

However, said former injection devices were not necessarily compatible with sensitive drugs such as biotech drugs and/or vaccines.

Besides, these documents do not address the issue of providing a coating that would keep its integrity within time and that would not have any, or at least any significant or adverse, interaction with the drug contained inside the injection device.

The document EP 1 060 031 describes a lubricated medical container that exhibits good gliding properties as well as low protein adsorption on the inner surface of the container.

Nevertheless, this document does not disclose any coating compatible with sensitive drugs such as vaccines and/or biotech drugs.

Indeed, as the coating described is not directed to be used with high-value drugs, even if the adsorption may be avoided, some denaturing of the drugs could occur.

This would be the case for example, with some polymers described in this document such as phosphorylcholine, 2-hydroxymethacrylate.

Additionally, this document is not directed to the integrity of the coating over time, after a long storage period.

However, the issue of storage is essential for prefilled syringes, as the coating needs to be stable over time not only before the filling of the drug in the injection device but also after the drug has been placed in the injection device.

Document US 6,461,334 also addresses the problem of drug adsorption on the coating and provides either a silicone coating crosslinked by irradiation or a silicone copolymer coating rendered hydrophilic by a plasma treatment.

Alternatively, document JP 2005-160888 provides a polyparaxylylene coating on the stopper to avoid drug adsorption on the stopper.

Document WO 2011/092536 discloses a medical injection device comprising a barrel and a stopper in gliding engagement within the barrel, wherein the inner volume of the barrel is composed of a distal portion containing a drug and a proximal portion containing a propelling fluid, said distal and proximal portions having different surface conditions and the drug being isolated from the piston and the proximal region where it slides.

The surface conditions of said distal and proximal portions are thus able to address different objectives, which may be incompatible.

On the one hand, the distal portion may be coated with a coating suited for drug stability.

In particular, said coating may be a hydrophilic coating, e.g. a hydrophilic polymer, possibly covering a hydrophobic pre-coating, e.g. PDMS, so as to obtain a barrier effect.

On the other hand, the proximal portion may be coated with a coating suited for a smooth gliding of the stopper.

In particular, said coating may be made of plasma-treated silicone.

Due to the isolation between the distal and proximal portions of the barrel, the coating of the proximal portion cannot be in contact with the drug contained in the distal portion; said coating can thus be chosen among coatings that would be likely to interact with the drug.

Recently, a new problem was raised by specialists of biotech drugs, which is the presence of particles in the pharmaceutical composition that could appear after the filling of the container, due to interaction of the drug with the coating present on the inner walls.

Indeed, these specialists have observed that significant amounts of subvisible particles (i.e. particles having a size comprised between 0.1 µm and 100 µm), in particular protein aggregates, in the pharmaceutical solution may be hazardous for the patients and they thus recommend that the level of particles in this size range be carefully controlled.

One can refer in this respect to "Overlooking Subvisible Particles in Therapeutic Protein Products: Gaps That May Compromise Product Quality", by John F. Carpenter et al., Journal of Pharmaceutical Sciences, Vol. 98, No 4, April 2009.

These recommendations result in more severe norms with regard to the maximum authorized particle level defined by the United States Pharmacopeia (USP 31 <788>) and the European Pharmacopeia (EP 6 <2.9.19>).

These norms not only place a heavy burden onto the pharmaceutical companies themselves but also onto the manufacturers of injection devices, since the release of particles from the containers into the pharmaceutical composition has to be avoided.

Document WO 2010/092536 discloses a pyrolyzing technique for forming a coating in a medical injection device in order to improve the gliding of the stopper.

However, this document does not address the above-mentioned problem of particles.

US 2008/071228 A1 discloses a similar method as defined in the preamble of claim 1.

It is thus necessary to provide a medical injection device that exhibits a good compatibility with sensitive drugs stored inside for long time period in order to meet the pharmacopeia norms with regard to the level of particles in the drug, while still providing good performance with regard to the gliding of the stopper within the barrel, and where the lubrication layer keeps its integrity over time.

A goal of the invention is to provide such injection devices.

### BRIEF DESCRIPTION OF THE INVENTION

An object of the invention is the use of plasma treated silicone oil as a coating in a medical injection device comprising a barrel and a stopper in gliding engagement within the barrel, for limiting the number of particles present on the surface of the coating and for limiting the number of particles released in a pharmaceutical composition contained in the injection device.

The present invention relates to a method of reducing a number of particles present on a surface of a coating or a number of particles released into a pharmaceutical composition contained in a medical injection device, according to the appended claims.

At least part of the pharmaceutical composition contained in said medical injection device is in contact with said coating.

According to an advantageous embodiment, the coating consists of crosslinked silicone.

According to an advantageous embodiment, said use is for increasing the gliding force of the stopper within the barrel as compared to a medical injection device having a not plasma-treated silicone coating.

The thickness of the coating is advantageously comprised between 90 and 400 nm.

The gliding force of the stopper is comprised between 1 and 8 N, preferably between 1.5 and 6 N.

According to an embodiment, the barrel is made of glass.

According to an embodiment, the silicone oil consists of 900 to 1200-centistoke polydimethylsiloxane (PDMS), preferably 1000-centistoke polydimethylsiloxane.

According to an embodiment, the injection device is a syringe having an internal volume of 1 ml and a diameter of 6.35 mm.

According to an embodiment, the weight of the coating is of between 0.1 and 0.4 mg.

According to an embodiment, the injection device is a prefilled syringe.

According to an embodiment, the pharmaceutical composition contained in the injection device is a biotech drug.

A further object of the disclosure is a medical injection device having:
a barrel having an inner surface;
a stopper in gliding engagement with the barrel; and
a pharmaceutical composition within the medical injection device and in contact with the inner surface of the barrel, the inner surface of the barrel comprising a coating of plasma treated silicone oil in contact with the composition,
wherein the plasma treatment of silicone oil reduces the number of particles present on the surface of the coating and the number of particles released into the pharmaceutical composition contained in the medical injection device as compared to silicone oil that is not plasma treated.

According to advantageous embodiments of said device, that may be considered independently or in combination:
- the particle level released in a solution contained within the medical injection device is less than 2.11 particles per mm² of the barrel surface in contact with the solution when measured by light obscuration method;
- the particle level released in a solution contained within the medical injection device is less than 10.56 particles per mm² of the barrel surface in contact with the solution when measured by microflow imaging method;
- said solution contained within the medical injection device is a mixture of Phosphate Buffered Saline and filtered Tween 80;
- the stopper is coated with silicone oil;
- the plasma treated silicone oil coating comprises a thickness between 90 and 400 nm;
- the plasma treated silicone oil coating increases a gliding force for moving the stopper within the barrel;
- a gliding force for moving the stopper within the barrel is between 1 and 8 N, more preferably said gliding force is between 1.5 and 6 N;
- the barrel is made of glass;
- the silicone oil comprises polydimethylsiloxane (PDMS);
- the silicone oil comprises a viscosity between 900 and 1200 centistoke;
- the medical injection device is a syringe having an internal volume of 1 mL and a diameter of 6.35 mm;
- the plasma treated silicone oil coating comprises a weight between 0.1 and 0.4 mg;
- the device is filled with said pharmaceutical composition to allow long term storage of the pharmaceutical composition meeting pharmacopeia norms with regard to the level of particles in the pharmaceutical composition;
- the device comprises a biotech drug within the barrel;
- the device comprises one or more of a protein, a peptide, a vaccine, DNA, or RNA within the barrel;
- the particles present on the surface of the coating and/or the particles released into the pharmaceutical composition contained in the medical injection device have a size ranging between approximately 0.1 µm and 100 µm.

A further object of the disclosure is a method of treating a medical injection device, the device comprising a barrel having a coated inner surface, a stopper in gliding engagement with the barrel, and, comprising:
(1) coating the inner surface of the barrel with a layer of a silicone oil; and
(2) exposing the silicone oil layer to a plasma treatment, thereby reducing the number of particles present on a surface of the coating or the number of particles released into a pharmaceutical composition contained in the medical injection device when the pharmaceutical composition is in contact with the inner surface of the barrel, as compared to a device having a barrel coated with silicone oil that is not plasma treated.

According to embodiments of either of those methods, that may be considered alone or in combination:
- the stopper is coated with silicone oil and the silicone oil coated stopper is exposed to a plasma treatment;
- the plasma treated silicone oil coating comprises a thickness between 90 and 400 nm;
- the plasma treated silicone oil coating increases a gliding force for moving the stopper within the barrel;
- the gliding force for moving the stopper within the barrel is between 1 and 8 N, preferably, said gliding force is between 1.5 and 6 N;
- the barrel is made of glass;
- the silicone oil comprises polydimethylsiloxane (PDMS);
- the silicone oil comprises a viscosity between 900 and 1200 centistoke;
- the pre-filled medical injection device is a syringe having an internal volume of 1 mL and a diameter of 6.35 mm;
- the plasma treated silicone oil coating comprises a weight between 0.1 and 0.4 mg;
- the device is filled with said pharmaceutical composition to allow long term storage of the pharmaceutical composition meeting pharmacopeia norms with regard to the level of particles in the pharmaceutical composition;
- the composition within the device is a biotech drug;
- the composition within the device is one or more of a protein, a peptide, a vaccine, DNA, or RNA;
- the plasma treated silicone oil coating comprises a cross-linked silicone;
- at least 90% of a surface of the inner surface of the barrel is coated with silicone oil;
- the plasma is produced by radio-frequencies;
- the plasma treatment comprises an oxidative plasma treatment performed under a mixture of oxygen and argon;
- the particle level released in a solution contained within the medical injection device is less than 2.11 particles per mm² of the barrel surface in contact with the solution when measured by light obscuration method;
- the particle level released in a solution contained within the medical injection device is less than 10.56 particles per mm² of the barrel surface in contact with the solution when measured by microflow imaging method;
- preferably, said solution contained within the medical injection device is a mixture of Phosphate Buffered Saline and filtered Tween 80;
- the particles present on the surface of the coating and/or the particles released into the pharmaceutical composition contained in the medical injection device have a size ranging between approximately 0.1 µm and 100 µm.

### BRIEF DESCRIPTION OF THE DRAWINGS

Other features and advantages of the invention will become apparent from the detailed description to follow, with reference to the appended drawings, in which:
- Figure 1 is a schematic sectional view of a medical injection device according to the invention;
- Figures 2A and 2B are schematic drawings of the light obscuration measurement (also referred to herein as the "HIAC" measurement, named after the brand of equipment manufactured by Hach Lange);
- Figures 3A and 3B are schematic drawings of the micro-flow imaging "MFI" measurement;
- Figure 4 shows the reduction of the particles at the coating in function of the duration of the plasma treatment, measured by HIAC;
- Figure 5 shows the reduction of the particles at the coating in function of the duration of the plasma treatment, measured by MFI.
- Figure 6 shows the evolution of gliding force of the stopper after one month of storage.

### DETAILED DESCRIPTION OF THE INVENTION

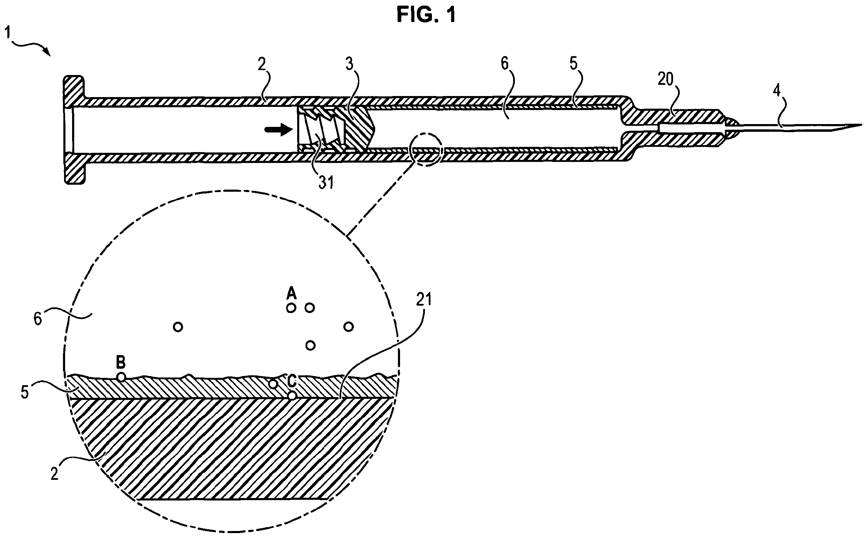

Referring to Figure 1, the injection device comprises a container, such as a barrel 2, and a stopper 3 in gliding engagement within the barrel 2.

The container 2 of the injection device may be made of any kind of glass or plastics suitable for medical applications.

The stopper 3 may be made of any elastomeric material, e.g. rubber, butyl rubber, silicone rubber.

The container 2 and /or the stopper 3 may be laminated with any suitable coating e.g. hydrophilic, hydrophobic coating as well as fluorinated coating.

The stopper 3 may be adapted to be connected to a plunger rod of a syringe or of an injection pump, for example.

To that end, it includes any suitable connecting means, e.g. a threaded portion 31, etc.

The end 20 of the barrel 2 opposed to the stopper 3 may be adapted to be connected to a needle 4, a cap or a catheter, or may comprise a staked needle.

The inner wall 21 of the barrel 2 is coated with a lubricant layer 5, which is a plasma-treated silicone layer.

The formation of the coating 5 according to an embodiment of the invention preferably comprises, in a first step, coating the inner walls of the barrel with silicone oil.

According to a preferred embodiment of the invention, the lubricant layer is sprayed onto the interior wall of the barrel 2.

To that end, a device comprising one or several nozzles may be introduced in the barrel 2 and moved along the barrel so as to uniformly deposit a silicone layer onto the inner wall of the barrel.

In a second step, the method comprises the exposure of the silicone oil layer to a plasma treatment.

To that end, the lubricated container is placed in a plasma reactor and plasma is generated in the reactor.

Numerous plasma processes are possible to achieve the plasma treatment.

However, in a preferred embodiment, the plasma treatment would be an oxidative plasma realized for example, under a mixture of oxygen and argon, such as 15% to 30% of oxygen and 30% to 70% of argon, preferably 25% of oxygen and 75% of argon.

The plasma may be produced by radio-frequency (i.e. with a frequency from 10 to 20 MHz, preferably from 11 to 14 MHz), with a power ranging from 50 W to 300 W, preferably from 150 to 220 W and under a vacuum of 10-100 mTorr in absolute value.

The exposure time of the coating is typically chosen to be between 1 second and 60 seconds, preferably from 10 to 40 seconds.

It should be clear to the skilled person that such parameters would be selected depending on the plasma reactor geometry, the volume of the injection device the arrangement of the injection devices inside the plasma reactor, the lubricant layer thickness, etc. in order to have the appropriate treatment according to the equipment used but also to the objects to be treated.

As a result of the plasma treatment, the silicone of the coating is crosslinked.

According to a preferred embodiment, the lubricant layer 5 is a silicone layer such as a polydimethylsiloxane (PDMS).

The viscosity of the silicone layer can be preferably comprised between 900 and 1200 centistoke.

More preferably, the lubricant layer is a 1000-centistoke PDMS.

The lubricant layer is applied so as to coat the major part of the interior wall 21 of the barrel.

Preferably, the lubricant coating 5 covers at least 90% of the interior surface of the barrel 2.

Preferably, the lubricant coating 5 has a thickness comprised between 90 nm and 400 nm, preferably about 150 nm.

According to an advantageous embodiment of the invention, the stopper is also lubricated in order to further reduce the gliding force.

In a preferred example, the stopper is a rubber stopper covered by a Flurotec^{®} barrier sold by West Pharmaceutical Services and further siliconized by PDMS DC-360.

Since the particles are induced by the interaction of the pharmaceutical composition 6 and the lubricant coating 5, there is a direct link between the number of particles and the surface area of the coating: the larger the surface of the coating is, the higher the amount of particles is.

It has been shown that with the coating according to an embodiment of the invention, the level of particles present in the solution 6 is below 2.11 particles by mm² of lubricant coating surface when measured by HIAC and below 10.56 particles by mm² of lubricant coating area when measured by MFI.

As shown on the enlarged view of Figure 1, during the life of a prefilled medical injection device, the presence of subvisible particles linked to the coating may raise three issues that are solved by the invention.

The first issue consists of silicone particles A (e.g. droplets) released into the pharmaceutical composition 6 and are thus present in the solution.

A second issue consists of aggregates or denatured drugs (referred to as B) that are present in the pharmaceutical solution 6 and that have been formed by the interaction of the pharmaceutical composition 6 and the silicone particles A.

A third issue consists of silicone particles (referred to as C) from the coating 5 that are released in the pharmaceutical solution 6 due to the mechanical friction of the stopper along the walls of the barrel.

With the plasma treated silicone layer according to an embodiment of the invention, the level of particles present on the surface of the coating is significantly reduced, which implies that the level of subvisible particles that may be released into the pharmaceutical composition or be in contact with the pharmaceutical composition during the life of the injection device is reduced.

As a consequence, the risk of aggregation of proteins in the solution itself, due to the adsorption generated at the silicone droplets, as well as the risk of adsorption of proteins at the surface of the barrel, are significantly reduced.

Therefore, the coating of the prefilled injection device remains stable over time meaning that no or no more aggregation neither denaturing of the drugs occur due to potential interaction with the coating present on the surface of the container.

Besides, since silicone particles are likely to be released in a very small amount into the pharmaceutical composition, they do not generate any background noise that would be detrimental to the monitoring of protein aggregation.

The coating in the medical container in accordance with an embodiment of the present invention also shows favorable mechanical properties. For example, no delaminating or break-up of the coating occurs under strong movement (from for example, transportation), as well as under dramatic changes in temperature. The coating remains attached to the barrel and no element is removed from it.

A further effect in accordance with an embodiment of the present invention is that the above-mentioned technical effects i.e. good gliding properties and favorable mechanical properties, remains stable during a long period of time, for example from 12 to 24 months.

This is particularly important when the prefilled medical container is stored in different conditions and positions, transport over long distances and then finally used to inject a pharmaceutical composition into a patient after a long period of storage after being filled.

Another advantage of an embodiment of the invention is that no further chemical species is introduced in the medical container, since the only chemical consists in a classic silicone oil, already used and authorized for such use.

This diminishes the risk of unpredictable side effects.

With a particle level below 2.11 particles/mm² of surface of the barrel in contact with the drug if measured by HIAC and below 10.56 particles/mm² of surface of the barrel in contact with the drug if measured by MFI, the obtained medical injection device fulfills the above-mentioned requirements, i.e. a high compatibility with vaccines and biotech drugs, good gliding performance, favorable mechanical properties and maintaining these properties over a long period of time (e.g. 12 to 24 months).

When comparing the amount of particles present in a container having plasma-treated silicone versus a similar container comprising a silicone oil coating that is not plasma-treated, the above values correspond respectively to a reduction of particles of at least 70% (when measured by HIAC) and of at least 90% (when measured by MFI).

The experimental protocols for these measurements are described in detail below.

In a preferred embodiment, the gliding force, i.e. the force required to move the stopper 3 in the barrel 2 is increased of 0.75 to 1.0 N as compared to a similar container comprising a not plasma-treated silicone oil lubricant.

Even if increasing the gliding force is usually strongly avoided by the skilled person, the applicant was able to correlate this increase, together with the dramatic decrease in term of particle level, with the expected properties of the medical injection device.

As a consequence, the medical injection device in accordance with an embodiment of the invention may advantageously be used as a prefilled injection device, i.e. an injection device that is filled with a pharmaceutical composition before being sold or delivered to end-users.

By pharmaceutical composition is meant any fluid manufactured in the pharmaceutical industry and delivered to patients in a therapeutic or diagnostic purpose.

In particular, the pharmaceutical composition that fills the medical injection device according to the invention may be a drug containing proteins, e.g. a vaccine, immunoglobulin or any other biotech drug.

For example, the medical injection device is a prefilled syringe.

### Particle level measurement methods

To measure the level of particles present on the surface of the barrel, the injection device is filled with a solution and the level of particles released in said solution is measured.

The solution that is used is a mixture of 10 g/L of Phosphate Buffered Saline and 2.13 mg/L of Tween 80 filtered with a 0.22 microns Stericup^{™} filter.

Then, the solution is stored for 2 hours before being introduced in a syringe. Once the syringes are filled, they are stirring during 48 h and the measurement of the particles is realized.

Depending on the size of the particles to be detected and quantified, different methods and equipments may be used.

In accordance with an embodiment of the present invention, the particles that are quantified in the lubricated medical injection device have a size of between 1 µm and 100 µm, preferably between 1 µm and 10 µm.

With a size ranging from 1 to 100 µm, the particles are called "subvisible particles", whereas above 100 µm, the particles are called "visible particles".

For such particle sizes, the most suitable counting methods are based on optical technologies: a first method is Light Obscuration (LO), a second method is Micro Flow Imaging (MFI), both of which being described in detail below.

### Light Obscuration

Light obscuration is routinely used to detect and measure subvisible particles present in parenteral solutions.

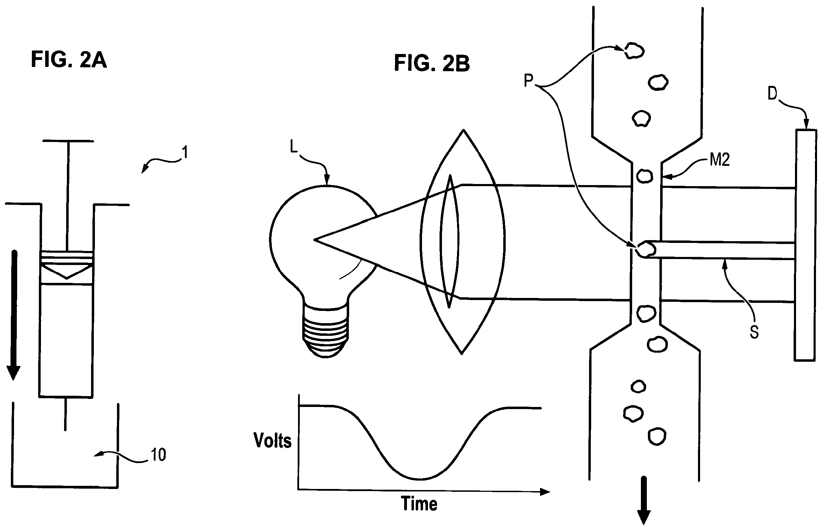

As illustrated on Figure 2B, when a particle P transits the measurement zone MZ in the direction of the arrow, it obscures (shadow S) an optical beam (e.g. generated by a light source L such as a laser diode with a wavelength of 680 nm), which results in a change in signal strength at a detector D.

This signal change is then equated to a particle's equivalent spherical diameter based on a calibration curve created using polystyrene spheres of a known size.

Devices based on this technique are sold under the brand HIAC by Hach Lange, for example.

Advantages of such light obscuration device are that it is easy to used, automated and fast.

The measured particles size range with such a device is typically comprised between 2 and 400 µm.

To provide good accuracy, the device has to be used with a large volume of solution (more than 3 ml, which is greater than the volume of a single 1 ml syringe), which implies that it does not allow analyzing containers one by one.

Therefore, as illustrated on Figure 2A, several devices 1 (only one is shown here) have to be flushed in an intermediate larger container 10 (e.g. a beaker) and the content of said intermediate container 10 is then analyzed by the light obscuration device.

The fluid used to carry out the particles level measurement is WFI (water for injection).

The protocol is the following.

The HIAC equipment is first cleaned with a mixture of WFI and isopropanol alcohol (50/50 proportion), then with WFI only.

All the glassware (intermediate containers for flushing the content of the containers that have to be tested) is also cleaned with WFI, so that the number of particles having a size of 10 µm is of less than 1 particle/ml.

Between each run, a blank with WFI is launched so as to check cell probe and glassware cleanliness.

Then the pharmacopeia norms are conducted on the WFI flushed from the containers. This means that the stopper is moved towards the distal direction in order to eject the WFI through the nozzle of the container into the intermediate container 10.

Usually, the program consists of four runs of 3 ml with the first run discarded, with 3 more ml in order to avoid air bubbles.

In the case of 1 ml injection device, 15 devices are flushed to a common recipient 10 in order to obtain the required analysis volume.

### Micro Flow imaging

MFI is a flow microscopic technology which operates by capturing images of suspended particles in a flowing stream.

Different magnification set-points are available to suit the desired size range and image quality.

The images are used to build a particle database including count, size, transparency and shape parameters.

Said database can be interrogated to produce particle size distributions and isolate subpopulations using any measured parameter.

Suitable equipments are for example sold by Brightwell Technologies (e.g. MFI DPA4200).

The solution is pumped from the injection tip of the container and goes through a flow cell.

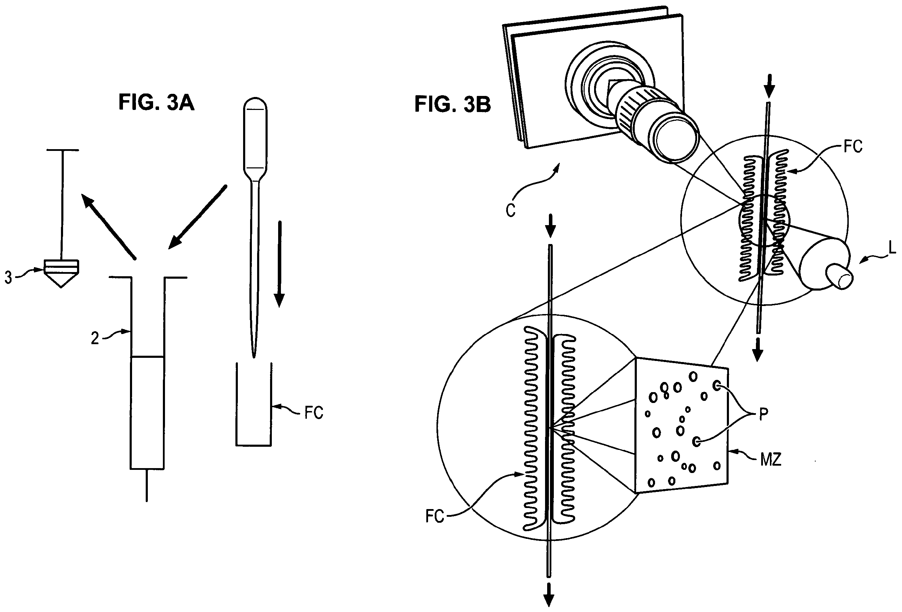

As illustrated on Figure 3B, a camera C acquires several pictures of a small zone MZ of the flow cell FC with a known frame rate.

On each picture, pixel contrast differences with calibrated background mean that there is a particle P.

The particle is then digitally imaged.

Supplementary characteristics are thus given (size, shape, etc.).

Due to the particles imaging, the advantage of this device is that it allows making the difference between an air bubble and a silicone oil droplet.

The measured particles size range is typically of between 1 and 100 µm.

The protocol is the following.

First, flow cell integrity is checked to ensure that the measures will be precise.

Then the cleanliness of the flow cell and the tubing is controlled by a blank with WFI (the particle number has to be below 100 particles/mL).

A run with certified beads (e.g. with a size of 5 or 10 µm and with a concentration of 3000 particles/ml) may be carried out.

The measurement program usually consists of 0.5 ml runs separated by 0.2 ml purge.

In this case, and unlike the analysis protocol used for Light Obscuration, several syringes are not flushed to a common recipient.

Instead, as shown on Figure 3A, the stopper 3 of each syringe to analyze is removed and 0.5 ml of solution is pipeted from the syringe 2 to the equipment.

A rinsing step is performed between the analyses of each syringe.

Depending on the selected method for counting the particles, the measured particle level may vary significantly.

According an embodiment of the present invention, the particle level when measured by HIAC as described above is of less than 2.11 particle/mm² of surface of contact between the barrel and the solution, whereas a measurement by MFI as described above indicates a particle level of less than 10.56 particle/mm² of surface of contact between the barrel and the solution.

### Gliding properties measurement

In order to evaluate the gliding forces, a 1 ml long glass syringe closed by a Daikyo Flurotec^{®} stopper is filled with WFI before being connected to a traction-compression bench (Lloyd LRX Plus) to induce gliding of the stopper within the container.

Then, in order to estimate the comportment of the coating during time, measurement of the gliding forces is performed on syringes coated with 0.25 mg of silicone submitted to plasma treatment at "T0" meaning immediately after the formation of the coating on the syringe surface, and at "T1" meaning after 1 month under 40 °C and 75 % of relative humidity.

In parallel, a visual check is performed to ensure that no delaminating or break-up occurred during the one month period.

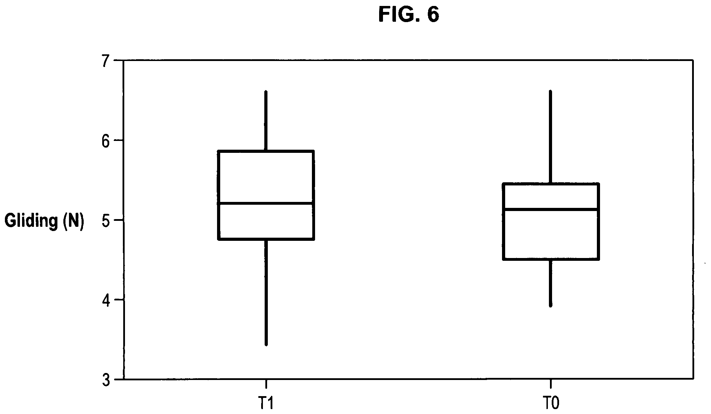

The results of such experiments can be seen on Figure 6, where the gliding forces immediately after plasma treatment are comprised between 3.9 N and 6.6 N while at T1 after one month, the gliding forces are between 3.4 and 6.6 N.

Therefore, it can be concluded that the plasma treated coating is not deteriorated with time as the gliding measurements remain in acceptable ranges, meaning between 1 N and 8 N.

### Experimental data

The following illustrates experimental data obtained on a medical container consisting of a 1 ml Long glass syringe.

For such a syringe, the barrel has an inner diameter of 6.35 mm.

The stopper was a rubber stopper with a Flurotec^{®} barrier and further siliconized with PDMS DC-360.

To obtain a silicone layer having a thickness of between 90 and 400 nm, with an average thickness of 150 nm, between 0.1 and 0.4 mg of silicone oil have been sprayed within the barrel with a spraying device.

The silicone oil used was 1000-ctsk DC-360 PDMS.

Then, a plasma treatment with a mixture of oxygen and argon was produced by radio-frequency, with a power ranging from 50 to 300 W and under a vacuum of 10-100 mTorr in absolute value.

The particles level has been measured both by HIAC light obscuration and by MFI method according to the experimental protocols described above.

With the first method, the particle level was of below 1500 particles/mL.

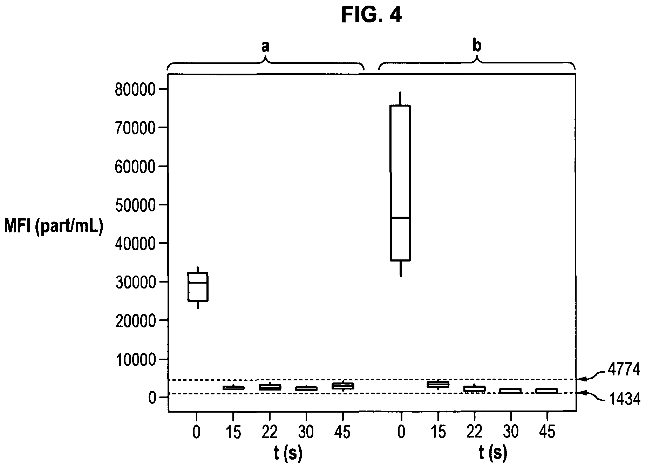

Figure 4 shows the reduction of the particle level on the coating in function of the duration of the plasma treatment, measured by HIAC.

Curve (a) corresponds to a weight of silicone of 0.25 mg, wherein curve (b) corresponds to a weight of silicone of 0.40 mg.

The greater the weight of silicone, the longer the plasma treatment has to be applied to reach the same particle level.

With the second method, the particle level was of below 7500 particles/ml and even below 4800 particles/mL.

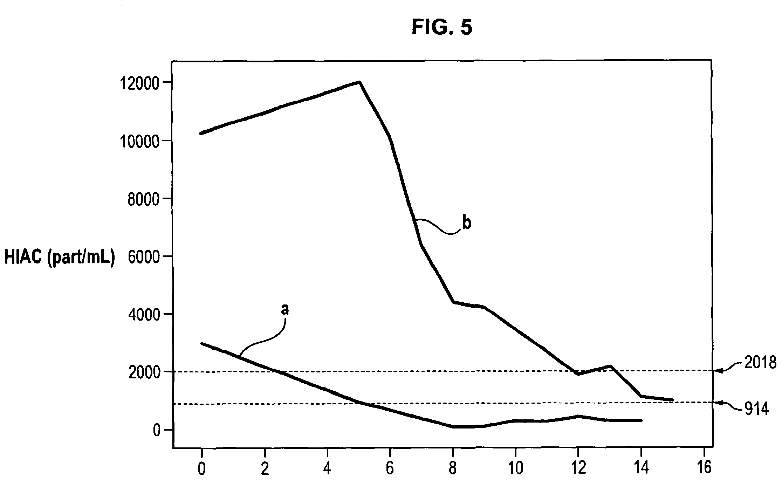

Figure 5 shows the reduction of the particles on the coating in function of the duration of the plasma treatment, measured by MFI.

Boxplots (a) corresponds to an injection device with a silicone coating of 0.15 mg, whereas boxplots (b) correspond to an injection device with a silicone coating of 0.40 mg.

Since the surface of the barrel in contact with the pharmaceutical composition is of about 710 mm², the particle levels correspond to less than 2,11 particles by mm² of coating and less than 10.56 particles by mm² of coating, respectively.

While specific embodiments of the invention are described with reference to the figures, those skilled in the art may make modifications and alterations to such embodiments without departing from the scope of the invention. Accordingly, the above detailed description is intended to be illustrative rather than restrictive. The invention is defined by the appended claims, and all changes to the invention that fall within the meaning and range of the claims are to be embraced within their scope.

## Claims

1. A method of reducing a number of particles present on a surface of a coating or a number of particles released into a pharmaceutical composition contained in a medical injection device, the device comprising a barrel having a coated inner surface, a stopper in gliding engagement with the barrel, the pharmaceutical composition being in contact with the inner surface of the barrel, said method **characterised in that**,
it further comprises, prior to disposing the pharmaceutical composition within the device, preparing the coating by:
(1) coating the inner surface of the barrel with a layer of polydimethylsiloxane (PDMS) with a viscosity between 900 and 1200 centistoke, said layer having a thickness between 90 and 400 nm; and
(2) exposing the polydimethylsiloxane layer to a plasma treatment, thereby reducing the number of particles present on the inner surface of the coating or reducing the number of particles released into the pharmaceutical composition contained in the medical injection device as compared to a device comprising a barrel coated with PDMS that is not plasma treated.

2. The method according to claim 1, wherein the stopper is coated with silicone oil and the silicone oil coated stopper is exposed to a plasma treatment.

3. The method according to claim 1, wherein the plasma treated polydimethylsiloxane coating increases a gliding force for moving the stopper within the barrel.

4. The method of claim 3, wherein the gliding force for moving the stopper within the barrel is between 1 and 8 N.

5. The method of claim 4, wherein the gliding force for moving the stopper within the barrel is between 1.5 and 6 N.

6. The method according to claim 1, wherein the barrel is made of glass.

7. The method according to claim 1, wherein the pre-filled medical injection device is a syringe having an internal volume of 1 mL and a diameter of 6.35 mm.

8. The method of claim 7, wherein the plasma treated PDMS coating comprises a weight between 0.1 and 0.4 mg.

9. The method according to claim 1, wherein the device is filled with said pharmaceutical composition to allow long term storage of the pharmaceutical composition meeting pharmacopeia norms with regard to the level of particles in the pharmaceutical composition.

10. The method according to claim 1, wherein the composition within the device is a biotech drug.

11. The method according to claim 1, wherein the composition within the device is one or more of a protein, a peptide, a vaccine, DNA, or RNA.

12. The method according to claim 1, wherein at least 90% of a surface of the inner surface of the barrel is coated with the polydimethylsiloxane.

13. The method according to claim 1, wherein the plasma is produced by radio-frequencies.

14. The method according to claim 1, wherein the plasma treatment comprises an oxidative plasma treatment performed under a mixture of oxygen and argon.

15. The method according to claim 1, wherein the particle level released in a solution contained within the medical injection device is less than 2.11 particles per mm² of the barrel surface in contact with the solution when measured by light obscuration method.

16. The method according to claim 1, wherein the particle level released in a solution contained within the medical injection device is less than 10.56 particles per mm² of the barrel surface in contact with the solution when measured by microflow imaging method.

17. The method according to claim 1, wherein the solution contained within the medical injection device is a mixture of Phosphate Buffered Saline and filtered Tween 80.

18. The method according to claim 1, wherein the particles present on the surface of the coating and/or the particles released into the pharmaceutical composition contained in the medical injection device have a size ranging between approximately 0.1 µm and 100 µm.

## Patentansprüche

1. Verfahren zum Vermindern einer Anzahl von Partikeln, die auf einer Oberfläche einer Beschichtung vorhanden sind, oder einer Anzahl von Partikeln, die in eine pharmazeutische Zusammensetzung freigesetzt werden, die in einer medizinischen Injektionsvorrichtung enthalten ist, wobei die Vorrichtung einen Zylinder, der eine beschichtete innere Oberfläche aufweist, und einen Stopper in gleitendem Eingriff mit dem Zylinder umfasst, wobei die pharmazeutische Zusammensetzung mit der inneren Oberfläche des Zylinders in Kontakt ist, wobei das Verfahren **dadurch gekennzeichnet ist, dass** es vor dem Anordnen der pharmazeutischen Zusammensetzung innerhalb der Vorrichtung ferner das Herstellen der Beschichtung durch Folgendes umfasst:
(1) Beschichten der inneren Oberfläche des Zylinders mit einer Schicht aus Polydimethylsiloxan (PDMS) mit einer Viskosität von zwischen 900 und 1200 Centistokes, wobei die Schicht eine Dicke von zwischen 90 und 400 nm aufweist; und
(2) Aussetzen der Polydimethylsiloxanschicht gegenüber einer Plasmabehandlung, wodurch die Anzahl von Teilchen, die auf der inneren Oberfläche der Beschichtung vorhanden sind, vermindert wird oder die Anzahl von Teilchen, die in die pharmazeutische Zusammensetzung freigesetzt werden, die in der medizinischen Injektionsvorrichtung enthalten sind, im Vergleich zu einer Vorrichtung vermindert wird, die einen mit PDMS beschichteten Zylinder umfasst, der nicht plasmabehandelt ist.

2. Verfahren nach Anspruch 1, wobei der Stopper mit Silikonöl beschichtet wird und der mit Silikonöl beschichtete Stopper einer Plasmabehandlung ausgesetzt wird.

3. Verfahren nach Anspruch 1, wobei die plasmabehandelte Polydimethylsiloxanbeschichtung eine Gleitkraft zum Bewegen des Stoppers innerhalb des Zylinders erhöht.

4. Verfahren nach Anspruch 3, wobei die Gleitkraft zum Bewegen des Stoppers innerhalb des Zylinders zwischen 1 und 8 N beträgt.

5. Verfahren nach Anspruch 4, wobei die Gleitkraft zum Bewegen des Stoppers innerhalb des Zylinders zwischen 1,5 und 6 N beträgt.

6. Verfahren nach Anspruch 1, wobei der Zylinder aus Glas besteht.

7. Verfahren nach Anspruch 1, wobei die vorgefüllte medizinische Injektionsvorrichtung eine Spritze ist, die ein Innenvolumen von 1 ml und einen Durchmesser von 6,35 mm aufweist.

8. Verfahren nach Anspruch 7, wobei die plasmabehandelte PDMS-Beschichtung ein Gewicht von zwischen 0,1 und 0,4 mg umfasst.

9. Verfahren nach Anspruch 1, wobei die Vorrichtung mit der pharmazeutischen Zusammensetzung gefüllt wird, um eine Langzeitlagerung der pharmazeutischen Zusammensetzung zu erlauben, die Arzneibuchstandards hinsichtlich des Gehalts an Partikeln in der pharmazeutischen Zusammensetzung erfüllt.

10. Verfahren nach Anspruch 1, wobei die Zusammensetzung innerhalb der Vorrichtung ein Biotech-Arzneimittel ist.

11. Verfahren nach Anspruch 1, wobei die Zusammensetzung innerhalb der Vorrichtung eines oder mehrere von einem Protein, einem Peptid, einem Impfstoff, DNA oder RNA ist.

12. Verfahren nach Anspruch 1, wobei mindestens 90 % einer Oberfläche der inneren Oberfläche des Zylinders mit dem Polydimethylsiloxan beschichtet ist.

13. Verfahren nach Anspruch 1, wobei das Plasma durch Hochfrequenzen hergestellt wird.

14. Verfahren nach Anspruch 1, wobei die Plasmabehandlung eine oxidative Plasmabehandlung umfasst, die unter einem Gemisch aus Sauerstoff und Argon durchgeführt wird.

15. Verfahren nach Anspruch 1, wobei der Partikelgehalt, der in eine Lösung freigesetzt wird, die innerhalb der medizinischen Injektionsvorrichtung enthalten ist, kleiner als 2,11 Partikel pro mm² der Zylinderoberfläche im Kontakt mit der Lösung, gemessen durch Lichtabschattungsverfahren, ist.

16. Verfahren nach Anspruch 1, wobei der Partikelgehalt, der in eine Lösung freigesetzt wird, die innerhalb der medizinischen Injektionsvorrichtung enthalten ist, kleiner als 10,56 Partikel pro mm² der Zylinderoberfläche im Kontakt mit der Lösung, gemessen durch Microflow-Imaging-Verfahren, ist.

17. Verfahren nach Anspruch 1, wobei die Lösung, die in der medizinischen Injektionsvorrichtung enthalten ist, ein Gemisch aus phosphatgepufferter Salzlösung und gefiltertem Tween 80 ist.

18. Verfahren nach Anspruch 1, wobei die Partikel, die auf der Oberfläche der Beschichtung vorhanden sind, und/oder die Partikel, die in die pharmazeutische Zusammensetzung freigesetzt werden, die in der medizinischen Injektionsvorrichtung enthalten ist, eine Größe von zwischen ungefähr 0,1 µm und 100 µm aufweisen.

## Revendications

1. Procédé de réduction du nombre de particules présentes sur une surface d'un revêtement ou du nombre de particules libérées dans une composition pharmaceutique contenue dans un dispositif d'injection médical, le dispositif comprenant un cylindre ayant une surface intérieure revêtue, un bouchon en prise glissante avec le cylindre, la composition pharmaceutique étant en contact avec la surface intérieure du cylindre, ledit procédé étant **caractérisé en ce qu'**il comprend en outre, avant de disposer la composition pharmaceutique à l'intérieur du dispositif, la préparation du revêtement par :
(1) le revêtement de la surface interne du cylindre avec une couche de polydiméthylsiloxane (PDMS) ayant une viscosité comprise entre 900 et 1200 centistokes, ladite couche ayant une épaisseur comprise entre 90 et 400 nm ; et
(2) l'exposition de la couche de polydiméthylsiloxane à un traitement au plasma, réduisant ainsi le nombre de particules présentes sur la surface interne du revêtement ou réduisant le nombre de particules libérées dans la composition pharmaceutique contenue dans le dispositif d'injection médical par rapport à un dispositif comprenant un cylindre revêtu de PDMS qui n'est pas traité au plasma.

2. Procédé selon la revendication 1, dans lequel le bouchon est revêtu d'huile de silicone et le bouchon revêtu d'huile de silicone est exposé à un traitement au plasma.

3. Procédé selon la revendication 1, dans lequel le revêtement en polydiméthylsiloxane traité au plasma augmente la force de glissement pour déplacer le bouchon à l'intérieur du cylindre.

4. Procédé de la revendication 3, dans lequel la force de glissement pour déplacer le bouchon à l'intérieur du cylindre est comprise entre 1 et 8 N.

5. Procédé de la revendication 4, dans lequel la force de glissement pour déplacer le bouchon à l'intérieur du cylindre est comprise entre 1,5 et 6 N.

6. Procédé selon la revendication 1, dans lequel le cylindre est en verre.

7. Procédé selon la revendication 1, dans lequel le dispositif médical d'injection pré-rempli est une seringue ayant un volume interne de 1 mL et un diamètre de 6,35 mm.

8. Procédé de la revendication 7, dans lequel le revêtement de PDMS traité par plasma comprend un poids compris entre 0,1 et 0,4 mg.

9. Procédé selon la revendication 1, dans lequel le dispositif est rempli de ladite composition pharmaceutique pour permettre un stockage à long terme de la composition pharmaceutique répondant aux normes de la pharmacopée en ce qui concerne le niveau de particules dans la composition pharmaceutique.

10. Procédé selon la revendication 1, dans lequel la composition à l'intérieur du dispositif est un médicament biotechnologique.

11. Procédé selon la revendication 1, dans lequel la composition à l'intérieur du dispositif est une ou plusieurs parmi une protéine, un peptide, un vaccin, de l'ADN ou de l'ARN.

12. Procédé selon la revendication 1, dans lequel au moins 90 % d'une surface de la surface interne du cylindre est revêtue du polydiméthylsiloxane.

13. Procédé selon la revendication 1, dans lequel le plasma est produit par des radiofréquences.

14. Procédé selon la revendication 1, dans lequel le traitement au plasma comprend un traitement au plasma oxydant réalisé sous un mélange d'oxygène et d'argon.

15. Procédé selon la revendication 1, dans lequel le niveau de particules libérées dans une solution contenue dans le dispositif d'injection médical est inférieur à 2,11 particules par mm² de la surface du cylindre en contact avec la solution lorsqu'il est mesuré par la méthode d'obscurcissement de la lumière.

16. Procédé selon la revendication 1, dans lequel le niveau de particules libérées dans une solution contenue dans le dispositif d'injection médical est inférieur à 10,56 particules par mm² de la surface du cylindre en contact avec la solution lorsqu'il est mesuré par la méthode d'imagerie par microécoulement.

17. Procédé selon la revendication 1, dans lequel la solution contenue dans le dispositif médical d'injection est un mélange de solution saline tamponnée au phosphate et de Tween 80 filtré.

18. Procédé selon la revendication 1, dans lequel les particules présentes à la surface du revêtement et/ou les particules libérées dans la composition pharmaceutique contenue dans le dispositif médical d'injection ont une taille comprise entre environ 0,1 µm et 100 µm.
